# EUROPEAN PATENT APPLICATION

(11) **EP 1 306 062 A1**
(43) Date of publication of application: **02.05.2003**
(21) Application number: 01650128.0
(22) Date of filing: 24.10.2001
(51) Int. Cl.: A61F 2/06, A61M 29/02

(54) **A catheter**

(71) Applicant: Moynesign Limited, Enniscorthy, Count Wexford (IE)
(72) Inventor: Carroll, Fiona Catherine, Charleville, County Cork (IE); Jowett, Mark, Enniscorthy, County Wexford (IE); Jones, Andrew, The Rower, County Kilkenny (IE); Kelly, Brian, Clifden, County Galway (IE)
(74) Representative: O'Brien, John Augustine

(57) **Abstract**

A peripheral catheter (1) has a stiff skived hypotube (5), a distal assembly (3) and a transition section (6) to connect the distal assembly (3) to the hypotube (5). A balloon (4) is mounted to the distal assembly (3) for use in an angioplasty procedure or during deployment of a stent. The catheter (1) has an inflation conduit (16) and a rapid exchange port (7) for receiving a rapid exchange guidewire. The distal assembly (3) comprises two coaxial distal tubes (14,15), which are spaced-apart to define therebetween a lumen (19) through which air for inflation of the balloon (4) is passed. The skive (8) of the hypotube extends through the transition section and substantially through the distal assembly, terminating just short of a shoulder of the balloon (4).

## Description

### Introduction

The invention relates to a catheter. In particular the invention relates to a peripheral catheter for use in carrying out an angioplasty and/or stenting of a stenosis in a renal artery.

There is a high incidence of renal artery stenosis in patients with coronary heart disease and general arteriosclerosis. Percutaneaous transluminal balloon dilation of a renal artery stenosis may be carried out. Artherosclerotic ostial lesions are generally also stented to prevent restenosis. Access to the renal artery transluminally however is difficult as the renal artery subtends an angle of 90° or greater to the aorta. This makes the angle of approach difficult and because of the angle of approach, there is reduced control over the end torque of the catheter. The proximal renal artery is unsupported and is one of the softest arteries in the body. Any rupture would result in massive haemorrhaging.

In general, a femoral artery is used as an access site and an 8F guiding catheter is advanced towards the entrance to the renal artery. Contrast media may be flushed through the catheter to assist in locating the entrance. A steerable guidewire is then advanced through the catheter and anchored in the kidney at the end of the renal artery. The guide catheter is removed and a balloon catheter is tracked along the guidewire to the site of the lesion.

It is also difficult to manipulate and control the balloon at the site of the stenosis. Because the clinician does not have adequate control of the catheter there is a tendency for the inflation of the balloon to cause the catheter to become dislodged from the ostium. The balloon may carry a stent. In some cases a pigtail catheter is introduced through a separate puncture site to introduce a contrast medium for monitoring the procedure at various stages.

Known systems for treating a stenosis in a renal artery therefore present a number of practical difficulties.

There is a need for an improved catheter which will overcome at least some of these problems.

### Statements of Invention

According to the invention there is provided a peripheral catheter comprising:-
a stiff shaft having a proximal end, a distal end, and a lumen extending axially therethrough;
a distal assembly having an inflation lumen and a guidewire lumen, the inflation lumen being in fluid communication with the lumen of the proximal shaft;
a balloon at the distal end of the catheter; and
a transition section extending between the distal region of the proximal shaft and the distal assembly;
the distal end of the shaft being skived and the skive of the shaft extending through the transition and substantially through the distal assembly.

Preferably the skive extends substantially to a distal bond to the balloon.

In a preferred embodiment the ratio of the length of the skived section of the stiff shaft to the unskived section of the stiff shaft is from 1:5 to 1:7, ideally approximately 1:6.5.

In one embodiment the transition section comprises:-
an inner transition portion located within the skive of the proximal shaft, the inner transition portion defining a transition lumen in fluid communication between the lumen of the shaft and the inflation lumen of the distal assembly; and
an outer transition portion located outside the distal region of the proximal shaft.

Preferably the inner portion of the transition section comprises a tube located within the distal region of the proximal shaft, the tube defining the transition lumen.

In one embodiment the outer portion of the transition section comprises a tube extending distally from a location proximal of the distal region of the proximal shaft.

Preferably inner and outer transition portions are fused together to form the fused transition section.

In a preferred embodiment the guidewire lumen is a rapid exchange guidewire lumen and the catheter includes a rapid exchange guidewire port. Ideally the skive of the proximal shaft extends distally of the rapid exchange port to provide support in the region of the rapid exchange port.

In a preferred embodiment the proximal shaft includes a guidewire lumen through which a guidewire may pass.

The proximal shaft is preferably a hypotube which may be of a metallic material, a polymeric material, or of a reinforced polymeric material.

In one embodiment the distal assembly is of a polymeric material. The transition section is also preferably of a polymeric material.

### Brief Description of Drawings

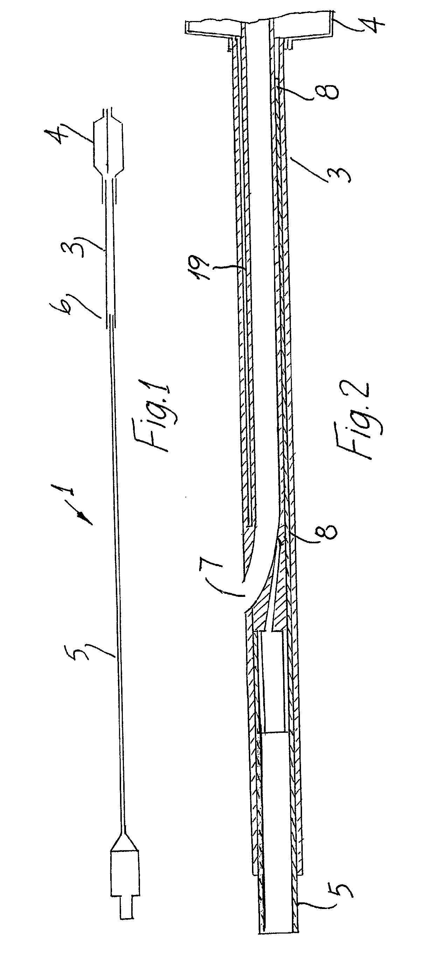

The invention will be more clearly understood from the following description thereof given by way of example only with reference to the accompanying drawings, in which:-
Fig. 1 is a schematic side view of a peripheral catheter according to the invention;
Fig. 2 is an enlarged cross sectional side view of a transition section of the catheter of Fig. 1;
Fig. 3 is an enlarged view of a proximal region of the transition section of Fig. 2;
Fig. 4 is a cross sectional view taken along the line F-F in Fig. 3;
Fig. 5 is a cross sectional view taken along the line E-E in Fig. 3;
Fig. 6 is a cross sectional view taken along the line D-D in Fig. 3;
Fig. 7 is an enlarged sectional view of a central region of the transition section of Fig. 2;
Fig. 8 is a cross sectional view taken along the line E-E in Fig. 7;
Fig. 9 is a cross sectional view taken along the line D-D in Fig. 7;
Fig. 10 is a cross sectional view taken along the line C-C in Fig. 7;
Fig. 11 is a cross sectional view taken along the line B-B in Fig. 7;
Fig. 12 is an enlarged cross sectional view of a distal region of the transition section of Fig. 2;
Fig. 13 is a cross sectional view taken along the line C-C in Fig. 12;
Fig. 14 is a cross sectional view taken along the line B-B in Fig. 12;
Fig. 15 is a cross sectional view taken along the line A-A in Fig. 12;
Figs. 16 to 18 are cross sectional side views illustrating assembly of the transition section of Fig. 2;
Fig. 19 is a side view of the transition section of Fig. 2 prior to bonding;
Fig. 20 and 21 are side views illustrating secondary bonding of the transition section of Fig. 2;
Fig. 22 is a side view of the transition section of Fig. 2 after bonding;
Fig. 23 is a cross sectional view taken along the line G-G in Fig. 22;
Figs. 24 and 25 are side views illustrating mating of the transition section and a distal assembly of the catheter prior to bonding;
Fig. 26 is a side view of the transition and the distal assembly after bonding;
Fig. 27 is a plan view of the transition section and the distal assembly after bonding;
Figs. 28 to 30 are schematic views illustrating the use of the catheter of the invention; and
Fig. 31 is an enlarged view of a distal end of the catheter, in use.

### Detailed Description

Referring to the drawings there is illustrated a peripheral catheter 1 according to the invention. The catheter 1 may used in treating a region of stenosis in a renal artery either by an angioplasty procedure or to deploy a stent.

The catheter 1 comprises a relatively stiff proximal shaft defined by a hypotube 5, a distal assembly 3 and a transition section 6 connecting the distal assembly 3 to the hypotube 5. A balloon 4 is mounted to the distal assembly 3 for use in any angioplasty procedure or for deployment of a stent.

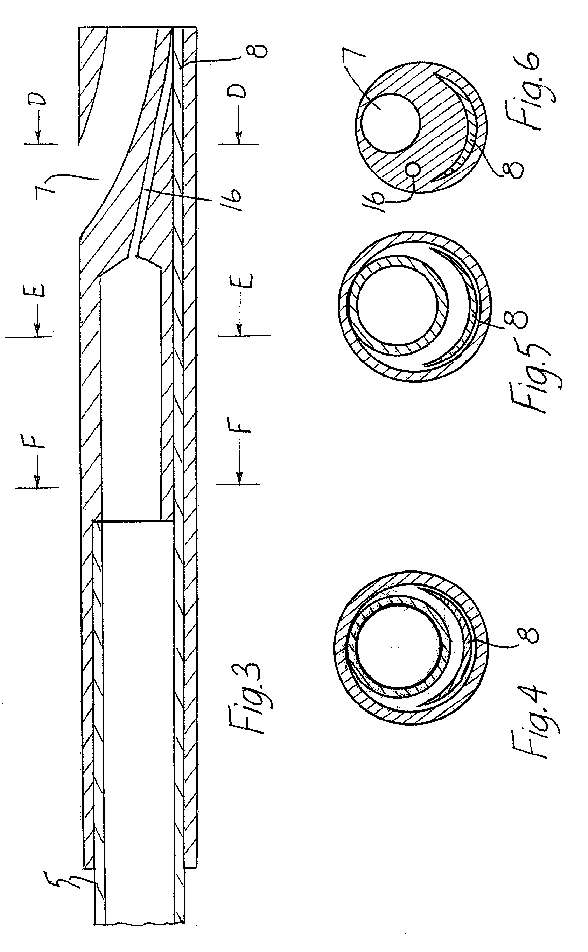

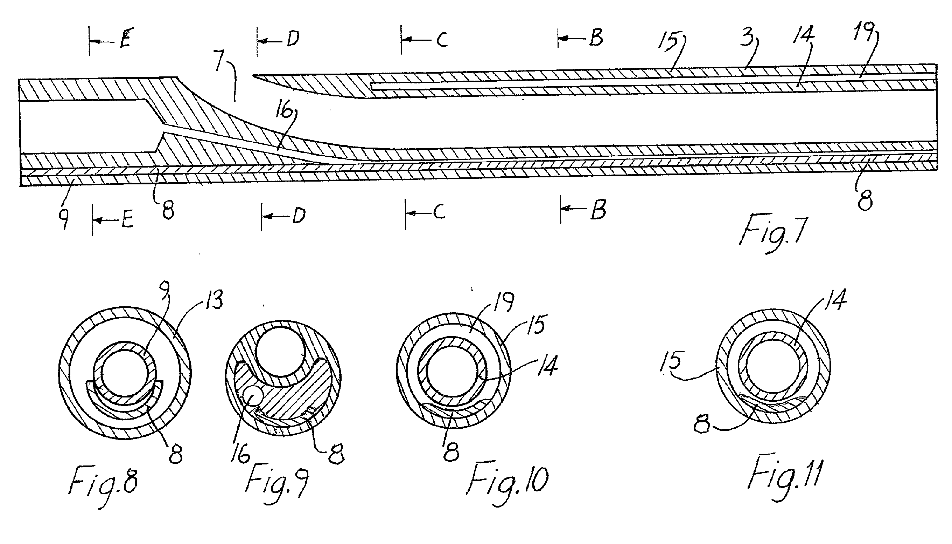

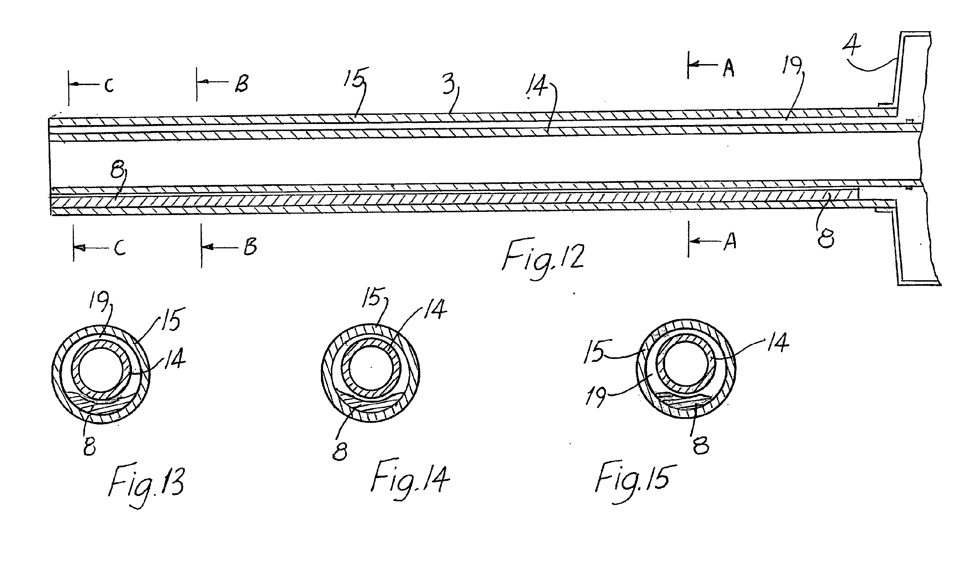

Referring in particular to Figs. 3 to 15 the transition section 6 is shown in detail. The side views in Figs. 3, 7 and 12 include gaps between the elements of the balloon catheter for ease of illustration. The cross sectional views in Figs. 4 to 6, 8 to 11 and 13 to 15 are a more accurate representation however of the relative dimensions of the catheter elements.

In this case the catheter 1 has a rapid exchange guidewire port 7 for receiving a rapid exchange guidewire (not shown).

The hypotube 5 is skived axially at its distal end to define a partially open distal region or skive 8 of the hypotube. On assembly, the hypotube skive 8 extends into the distal assembly 3 almost to the shoulder of the balloon 4.

The term skived as used in this specification refers to a hypotube with a tapered, sliced distal end to define a partially open distal region. The term bonding as used in this specification refers in particular but not exclusively to thermal bonding. The term bond refers in particular but not exclusively to an adhesive bond. The term bond also refers to a fused or encapsulated joint.

The transition section 6 has an inner portion defined by an inner tube 9 located within the distal region 8 of the hypotube 5 and an outer transition portion including a transition reinforcement tube 10 located outside the distal region 8 of the hypotube 5.

The inner tube 9 is located in the skive 8 intermediate the ends thereof. The transition reinforcement tube 10 extends from the main body of the hypotube 5, partially over the skive 8 and surrounds part of the inner tube 9.

The distal assembly 3 comprises inner and outer coaxial distal tubes 14, 15 which are spaced-apart to define therebetween a lumen 19 through which air for inflation of the balloon 4 is passed. The inner tube 14 is bent upwardly to define the rapid exchange port 7 at a proximal end of the distal assembly 3, and the rapid exchange guidewire (not shown) is passed through the inner tube 14.

The balloon catheter has an inflation conduit 16 for inflating and deflating the balloon 4. The inflation conduit 16 is defined by a hypotube lumen 30 and the inflation lumen 19 of the distal assembly which are interconnected by a transition lumen defined by the inner transition tube 9.

The inner tube 9, the transition reinforcement tube 10 and the outer reinforcement tube 13 are of a suitable thermoplastic material. For example, the distal inner and transition inner may be of a PEBAX material. The outer may be a blend of Nylon and a PEBAX.

The balloon catheter 1 is assembled in the following manner.

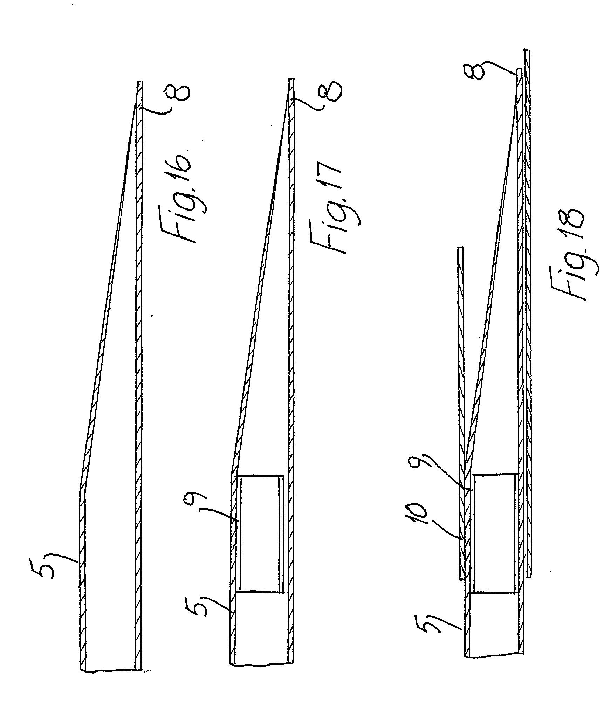

The hypotube 5 is skived at its distal end, as illustrated in Fig. 16, and the inner tube 9 is inserted into the skive 8, Fig. 17. Next the transition reinforcement tube 10 is positioned around the hypotube 5 and the inner tube 9.

In a primary bonding step the transition reinforcement tube 10 and the inner tube 9 are fused together into a mass 13 of polymeric tubing by heating. Portion of the skive 8 is thereby encapsulated with the fused transition.

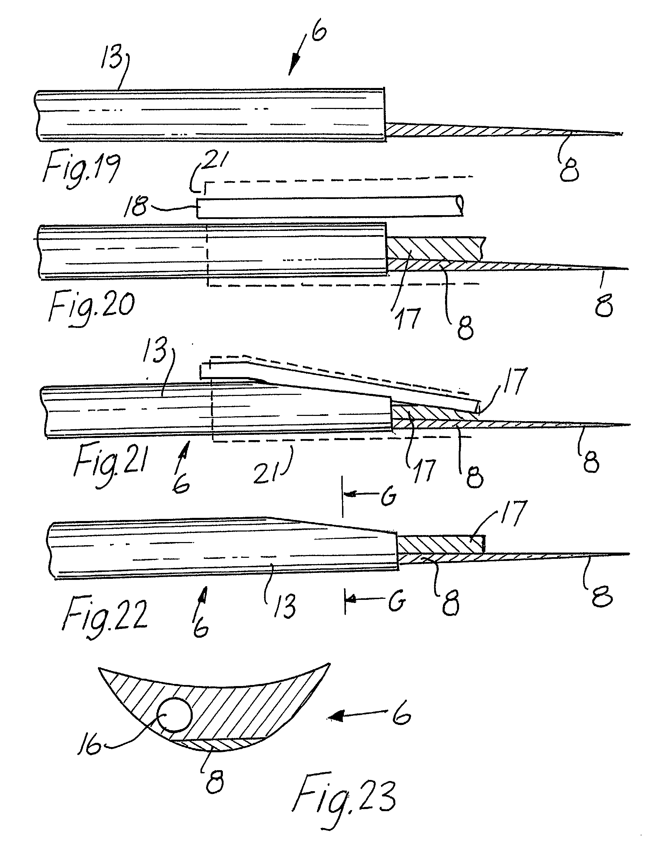

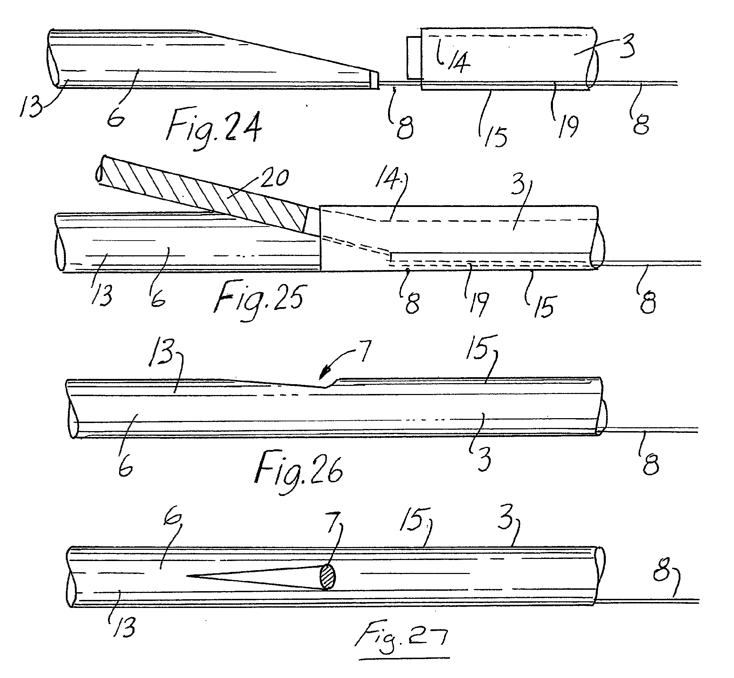

In a secondary bonding step, a first mandrel is inserted into the inner tube 9 from the distal end. A heat shrink tube 21 is positioned surrounding the distal end of the transition section 6, and a second mandrel 18 is positioned within the heat shrink tube 21 parallel to the hypotube 5, along the top of the transition section 6, as illustrated in Fig. 20. The transition assembly is fused together by heating it in the region of the distal end of the transition section 6. As the transition assembly fuses the contraction of the heat shrink tube 21 pushes the second mandrel 18 down fusing the inner tube 9 and the mass 13 together into a U-shaped cross section, Figs. 21 and 23. The skive 8 is encapsulated within the fused transition. After this secondary bonding step the heat shrink tube 21, which is removed after the secondary bonding stage, is of a suitable thermoplastic material, such as polytetrafluoroethylene (Teflon).

The distal assembly 3 is then positioned, such that its proximal end is adjacent and in line with the distal end of the transition section 6, and the two assemblies are mated together. On assembly the hypotube skive 8 and the first mandrel extend into the distal assembly 3. A third mandrel 20 is inserted into the proximal end opening of the inner distal tube 14, and moved, so that the inner distal tube 14 is bent upwardly to the top of the outer distal tube 15.

The mating of the assemblies is illustrated in Fig. 25. For convenience the first mandrel has been omitted from Fig. 25.

In a final bonding step, the mated assemblies are fused together by heating in the region of the proximal end of the distal assembly 3. The mandrels are then removed to define a rapid exchange port 7 (which enables access to the inner distal tube 14) and an inflation conduit 16 (for inflation of the balloon 4) respectively.

The invention allows a stiff hypotube to be securely attached to a relatively soft distal assembly with a smooth and effective transition. The large bonded mass at the transition prevent any kinking of the catheter, thus facilitating torque transmission and pushability.

The skive of the stiff shaft extends into the soft distal assembly to ensure a gradual change in catheter stiffness along the axial length of the catheter.

The skive of the stiff hypotube extends distally of the rapid exchange port to support the transition and the distal assembly in the region of the rapid exchange port.

The hypotube (including the skive) is preferably from 65 to 85cm long, usually about 75cm long. The skive extends for a distance of about 10 to 12cm, generally about 10cm. Thus, the ratio of the length of the skived section of the hypotube to the unskived section is from 1:5 to 1:7, preferably about 1:6.5.

The skive 8 of the hypotube extends through the transition and substantially through the distal assembly, stopping just short of a proximal shoulder of the balloon. In this way the transition and distal assembly are gradually reduced in stiffness. This provides torqueability right through to the shoulder of the balloon and allows the distal end of the catheter to be more easily manipulated and held in place during inflation of the balloon. Thus, the clinician is able to easily maintain the balloon in a desired position relative to the lesion. The dilation and/or stenting procedure is therefore more readily carried out.

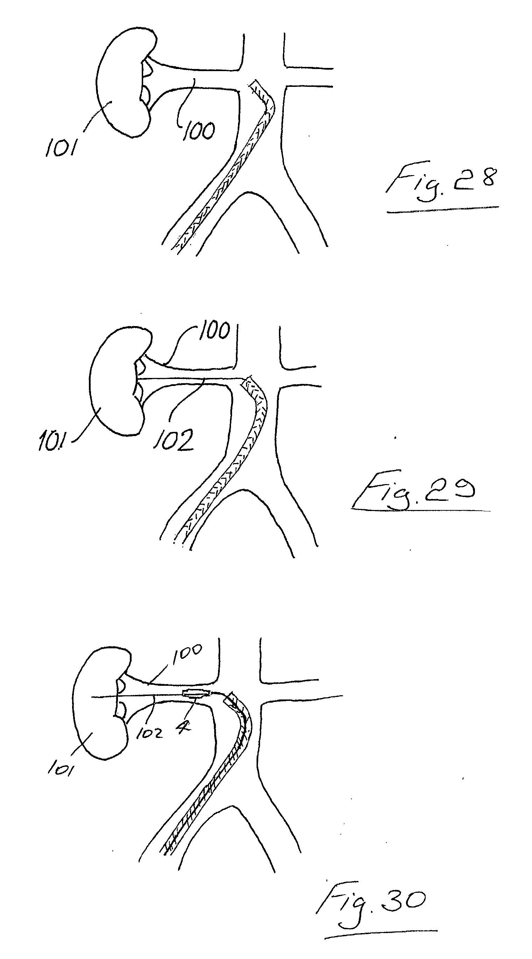

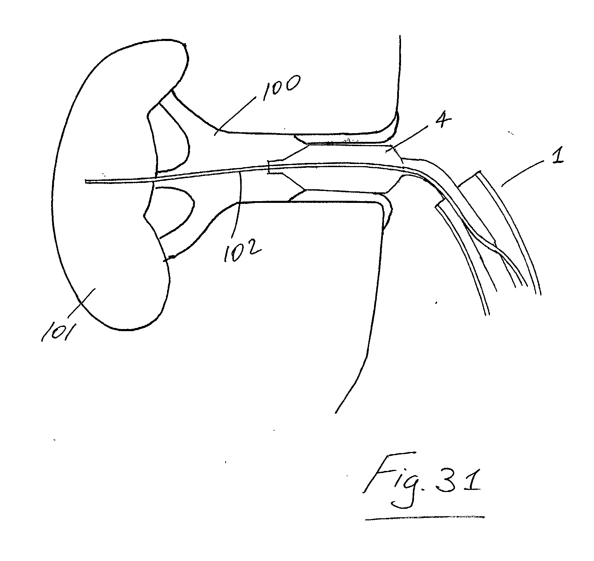

Referring to Figs. 28 to 31 there is illustrated schematically various steps in treating a stenosis in a renal artery 100 which terminates in a kidney 101. The catheter 1 is advanced from an entry site in a femoral artery towards the opening (ostium) into the renal artery 100 and the distal end of the catheter is turned by the clinician to allow a guidewire 102 to be delivered through the renal artery 100 and anchored in the kidney 101. The sheath of the catheter can be 5 Fr or less with a 0.018" guidewire providing a soft and flexible tip. The stiff proximal shaft of the hypotube provides the required torque and pushability to navigate the tortuous iliac. Because the distal tip section is short there is good control of positioning of the balloon over a lesion. The acute angle between the aorta and the renal artery can be overcome while allowing the distal end of the hypotube to reach the ostium. As a result, torque and pushability can be transmitted to the distal tip adjacent to the stenosis. The long skive provides the support and pushability to enable the balloon to enter the lesion. It eases the trackability over the lesion as the surgeon has good control. In addition, it gives rigidity at the beginning of the distal tip whilst providing flexibility at the balloon shoulder to negotiate the acute angle.

The rapid exchange port at the beginning of the skive provides the clinician with additional support for distal tip movement and balloon placement while providing support for movement of the guidewire.

The lesion is treated either using the balloon in a dilation procedure and/or for deploying a stent. Thereafter, the balloon is deflated and the catheter is withdrawn, followed by the guidewire.

The invention is not limited to the embodiments hereinbefore described which may be varied both in construction and in detail.

## Claims

1. A peripheral catheter comprising:-
a stiff shaft having a proximal end, a distal end, and a lumen extending axially therethrough;
a distal assembly having an inflation lumen and a guidewire lumen, the inflation lumen being in fluid communication with the lumen of the proximal shaft;
a balloon at the distal end of the catheter; and
a transition section extending between the distal region of the proximal shaft and the distal assembly;
the distal end of the shaft being skived and the skive of the shaft extending through the transition and substantially through the distal assembly.

2. A catheter as claimed in claim 1 wherein the skive extends substantially to a distal bond to the balloon.

3. A catheter as claimed in claim 1 or 2 wherein the ratio of the length of the skived section of the stiff shaft to the unskived section of the stiff shaft is from 1:5 to 1:7.

4. A catheter as claimed in claim 3 wherein the ratio is approximately 1:6.5.

5. A catheter as claimed in any preceding claim wherein the transition section comprises:-
an inner transition portion located within the skive of the proximal shaft, the inner transition portion defining a transition lumen in fluid communication between the lumen of the shaft and the inflation lumen of the distal assembly; and
an outer transition portion located outside the distal region of the proximal shaft.

6. A catheter as claimed in claim 5 wherein the inner portion of the transition section comprises a tube located within the distal region of the proximal shaft, the tube defining the transition lumen.

7. A catheter as claimed in claim 5 or 6 wherein the outer portion of the transition section comprises a tube extending distally from a location proximal of the distal region of the proximal shaft.

8. A catheter as claimed in any of claims 5 to 7 wherein the inner and outer transition portions are fused together to form the fused transition section.

9. A catheter as claimed in any preceding claim wherein the guidewire lumen is a rapid exchange guidewire lumen and the catheter includes a rapid exchange guidewire port.

10. A catheter as claimed in claim 9 wherein the skive of the proximal shaft extends distally of the rapid exchange port to provide support in the region of the rapid exchange port.

11. A catheter as claimed in any of claims 1 to 10 wherein the proximal shaft includes a guidewire lumen through which a guidewire may pass.

12. A catheter as claimed in any preceding claim wherein the proximal shaft is a hypotube which is selected from any one or more of a metallic material, a polymeric material or a reinforced polymeric material.

13. A catheter as claimed in any preceding claim wherein the distal assembly is of a polymeric material.

14. A catheter as claimed in any preceding claim wherein the transition section is of a polymeric material.
